# EUROPEAN PATENT SPECIFICATION

(11) **EP 0 790 766 B1**
(45) Date of publication and mention of the grant of the patent: **04.07.2001**
(21) Application number: 95932814.7
(22) Date of filing: 27.09.1995
(51) Int. Cl.: A01M 1/20, A01M 1/22, A61L 9/14

(54) **DISTRIBUTION DEVICE**
VERTEILUNGSVORRICHTUNG
DIFFUSEUR

(30) Priority: 01.10.1994 GB 9419815
(43) Date of publication of application: 27.08.1997
(73) Proprietor: UNIVERSITY OF SOUTHAMPTON, Southampton, Hampshire SO17 1BJ (GB)
(72) Inventor: HUGHES, John Farrell, Southampton SO40 2JL (GB); HOWSE, Philip Edwin, Gosport, Hants PO12 2NG (GB)
(74) Representative: Britter, Keith Palmer
(86) International application number: GB9502293
(87) International publication number: WO9610331

(56) References cited:
- EP-A- 0 243 031
- WO-A-82/00079
- DE-A- 3 332 449
- FR-A- 1 009 980
- US-A- 4 776 515
- US-A- 5 196 171

## Description

This invention relates to a distribution device for distributing biologically active materials into the atmosphere and a method and apparatus for trapping insects using the device.

Techniques for the liberation of chemicals into the air are well-known. They include the use of slow-release formulations, in which volatile compounds are released from surfaces, such as wicks, which retard or control the release rate, and the use of pressurised aerosol containers which offer an instant effect by manual activation of a pressure release valve.

Volatilisation rates of relatively involatile chemicals from an absorbent surface at ambient temperatures can be too low for many purposes. Aerosol containers can be used to atomise compounds resulting in high release rates. They are commonly used to distribute the atomised compounds within the confines of a room in order to achieve maximum effect of fragrance enhancement. The disadvantages are that the packaging of pressurised containers is expensive, they use large volumes of carrier gas, require filling under high pressure, the release rates are difficult to control, and the hydrocarbon gasses which are commonly used as propellants are environmentally damaging.

The zone of activity of a volatile attractant or repellant released from a point source, the so called "active space", is dependent upon the release rate of molecules from that source. Once released into the air above the boundary layer, volatilised attractants or repellants are dispersed mainly by air currents which can carry them for many metres as a coherent plume, before turbulence destroys the coherence. Animals, such as insects, can thus be attracted upwind in an odour plume from distances extending to kilometres in appropriate conditions. In conditions of still air, such as occurs in the boundary layer close to any solid substrate, dispersion is mainly by diffusion leading to a concentration gradient around the source. This concentration gradient can provide short-lived orientational cues to insects (and other animals), but usually only over distances of the order of a few centimetres. For effective distribution, a chemical compound or mixture must therefore be projected into the air above the boundary layer, where it will be carried further by the ambient air flow.

In EP-A-0243031, there is disclosed apparatus for electrostatically spraying liquids, such as pesticides, paints, lacquers, adhesives and release agents, on to a target, wherein a nozzle spraying edge has a plurality of sites which, in use, are covered by liquid to be sprayed. The local electric field strength can be intensified, such that the liquid at the sites is drawn out mainly by electrostatic forces into ligaments which eventually break up into electrically charged particles to be sprayed on to a target.

US-A-5196171 discloses apparatus for generating electrostatically-charged aerosols and vapours for aromatically conditioning a room or other enclosed space or for modifying and enhancing the quality of such a space using a liquid conditioning substance, for example, on aromatic oil, deodorant, disinfectant, fumigant, fungicide, insecticide or bactericide. An electrostatic charge is applied to a liquid supplied to a wick-like, porous emitter or generator assembly where an aerosol or vapour is generated in a controlled manner.

Essentially, each of these two prior art arrangements, and particularly the latter, could be described generally as a device for distributing biologically active material into the atmosphere, comprising a supply of such material to be so-distributed and means for vapourising, volatilising or atomising the material in aerosol form using an electrostatic technique, whereby the so-vapourised, volatilised or atomised material is suspended in the atmosphere.

Many insects attractants, including pheromones, are relatively involatile and therefore attract insects at only very short range. An example is the sexual pheromone of the common housefly, Musca domestica, which is commonly used in synthetic form ("muscalure" or (Z) -9-tricosene) to attract houseflies to traps or toxic baits. The volatility is such that attraction occurs only over a distance of a few centimetres. Similarly, many insect repellents are effective only at close range.

Thus, the present invention provides a device and a corresponding method for distributing biologically active material into the atmosphere which are distinguished from the prior art discussed above, in that the material to be so-distributed is a pheromone.

The range of attraction can be extended by releasing the pheromone into the air at a higher rate than occurs naturally so that it can be dispersed by air currents.

Preferably, the means for vapourising, volatilising or atomising the pheromone in aerosol form for subsequent atmospheric suspension, comprises means arranged to subject the pheromone to a sufficiently high electric field to cause the pheromone to become electrically charged to a sufficient extent to enable its atomisation in aerosol form into the atmosphere for subsequent suspension therein.

Preferably, the device further comprises a substrate which is capable of having an electrical potential applied thereto and upon which the pheromone to be vapourised, volatilised or atomised is receivable, such that the pheromone is electrically charged when subjected to the electric field generated by the electrical potential applied to the substrate.

The substrate may be in the form of a surface to which the pheromone to be vapourised, volatilised or atomised is supplied, such surface preferably being pointed to enhance the electrical field at its tip, when the electrical potential is applied.

Typically, the pointed substrate surface comprises one of an absorbent and a porous material.

The substrate may be electrically conductive or non-conductive and is preferably insulated from earth during use.

Alternatively, the pheromone to be vapourised, volatilised or atomised may be subject directly to the electric field, whether or not any substrate is used.

WO-A-82/00079 describes apparatus for dispersing a gaseous or vapourised pheromone trail into the atmosphere under determined conditions, the apparatus comprising a first container in which a pheromone source is located. Means above the first container are provided for creating an upward draught and flying insects lured by the trail enter the means and the first container and, thence, a second container from which they cannot escape. Alternatively, the insects land on a sticky insecticide from which they are unable to escape.

Accordingly, another aspect of the present invention resides in an insect trapping apparatus comprises a distribution device according to the first aspect of the invention and trapping means for trapping insects lured by the pheromone.

According to a further aspect of the present invention, a method of trapping insects comprises distributing a pheromone into the atmosphere in accordance with the invention, whereby a plume of pheromone is produced along which insects orientate; and providing trapping means at the source of the plume whereby insects are attracted by the pheromone and follow the plume to the trapping means to be trapped therein.

In a preferred embodiment, the inventive apparatus and method are used to vapourise, volatilise or atomise liquid pheromones, in which case, a pointed substrate surface, preferably of an absorbent or porous material, may be employed. In this arrangement, an electrical potential applied to the liquid and/or surface creates an electric field which results in the liquid being electrically charged. As a consequence, the so-charged ionic species migrate to the tip of the point where the electric field strength is at its greatest and a meniscus is formed in the region of the tip. Eventually, the charge density of the ions at the so-formed meniscus becomes so great that the external electric field causes them to overcome the surface tension at the meniscus at which disruption occurs, so that the ions can move freely and, hence, be released from the remainder of the liquid pheromone in the region of the meniscus as an aerosol of charged droplets and into the atmosphere.

The depth of penetration of the pheromone aerosol spray into the atmosphere will depend upon such factors as the electrical potential applied to the liquid pheromone and/or substrate, the electrical conductivity of the liquid pheromone and the surface tension thereof at its meniscus. Subsequent "evaporation" of the charged droplets into, say, molecules can also occur, with corresponding charge transfer. The electric field can be adjusted to control the rate at which the aerosol is generated and, also to switch it "on" and "off", if needs be.

The electrical charge may have either negative or positive polarity, and the level of charge may be determined by the electrical characteristics of the pheromone and the electrical energy input into the aerosol generating system. Liquid droplets or molecules that are atomised will, by virtue of their electrical charge, tend to interact with and become attached to airborne dust particles. This in turn imparts an electrical charge to any airborne dust within the aerosol treated area. Electrically charged particles are known to precipitate on to substrates more rapidly than uncharged particles.

The invention results in a more efficient and controllable method of releasing pheromones into the atmosphere for suspension therein. With the invention, vapourisation, volatilisation or atomisation of a pheromone, such as a liquid pheromone, may be achieved by electrostatic field enhancement. This offers a number of important advances over existing technology. In the first instance, no propellant gas is necessary. Secondly, the electrostatic release of the pheromone into the atmosphere offers more control over the aerosol characteristics. Thirdly, the aerosol created will carry an electrical charge and as a result will affect the distribution of dust particles in the air.

Further, the invention includes a controlled release device or method for releasing into the atmosphere in aerosol form pheromones for subsequent airborne suspension, which device or method incorporates any form of inventive apparatus or method defined above or otherwise described herein. This allows pheromone materials to be used to attract insects to a trap or ward them off a particular area without needing to disperse insecticides or other chemicals which could be harmful to humans or other animals. The insect trap of the present invention allows these harmful products to be restricted to the trap.

It is to be appreciated that the presently inventive apparatus and method are distinguished from known electrodeposition coating processes using electrostatic techniques, in that they release into the atmosphere a pheromone aerosol whose particles or droplets are subsequently suspended therein, whereas in known electrostatic deposition processes, particles are subsequently, and usually immediately, deposited on a substrate.

An example of a distribution device and trapping apparatus according to the present invention will now be described with reference to the accompanying drawings in which:
Figure 1 illustrates a distribution device according to the invention;
Figure 2 illustrates trapping apparatus incorporating the device of Figure 1;
Figure 3 is a graph of distribution of houseflies with time with no attractant present;
Figure 4 is a graph of distribution of houseflies with time with attractant introduced by conventional means; and,
Figure 5 is a graph of distribution of houseflies with time with attractant introduced using the device of Figure 1.

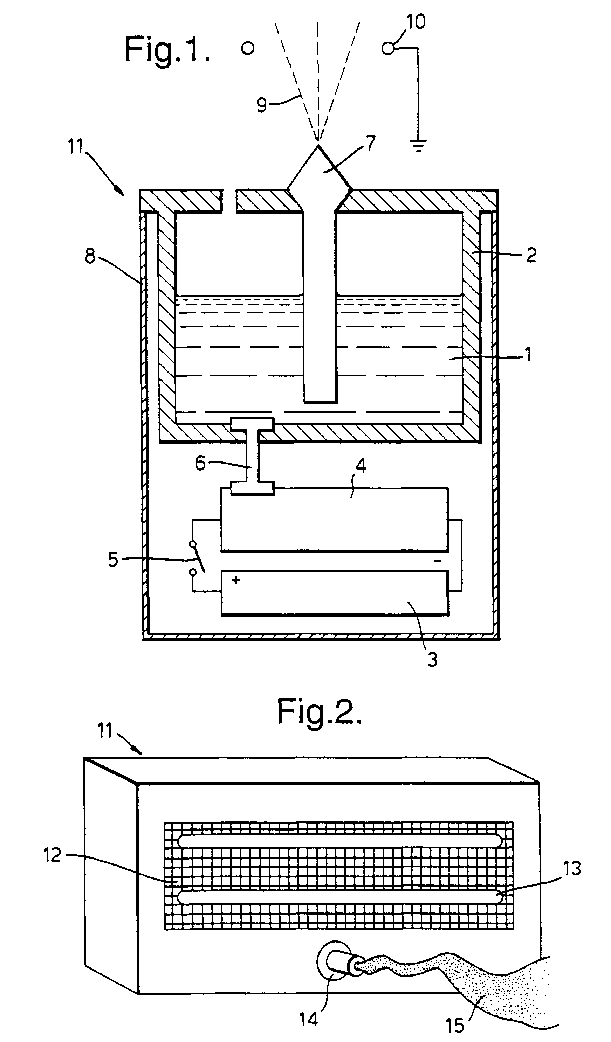

In the device of Figure 1, a supply of liquid pheromone 1 to be atomised is contained in a chamber 2 made of an electrically insulating material. The chamber 2 also contains a pointed wick 7 of absorbent or porous material, which is electrically insulated and which becomes saturated by the liquid pheromone 1 as a result of capillary action. The chamber 2 is located in a case 8 preferably also of electrically insulating material, which contains a battery 3 as a power supply, connected to a voltage multiplying circuit 4. Above the pointed wick 7 is an electrode 10, connected to ground.

A switch 5 actuates the power source battery 3 and an electrical contact 6 connects the voltage output to the liquid chamber 2. Subject to its electrical resistivity, the liquid pheromone 1 in the chamber 2 and within the wick 7 attains the same electrical potential as the voltage of the multiplying circuit 4. The electrode 10 is the reference point for the voltage output, so that a high electric field is produced between the pointed wick 7 and the electrode 10.

The liquid pheromone 1 becomes electrically charged and the so-charged liquid ions migrate to the tip of the wick 7 where the electric field strength is greatest, resulting in the formation of a liquid meniscus at the wick tip. The charge density of the ions at the so-formed meniscus becomes so great, due to the electric field enhancement thereat, that the electric field causes disruption and deformation of the meniscus so that the ions can move freely and, hence, overcome the surface tension of the liquid at the meniscus. As a result, the ions are released from the remainder of the liquid pheromone in the region of the meniscus at the wick tip as an aerosol spray of charged liquid droplets and into the atmosphere. Such atomisation of the liquid pheromone 1 into a fine aerosol spray 9 with each liquid droplet carrying an electric charge causes them to be suspended in the atmosphere. The charged liquid droplets move away from the wick tip. In the process, the charged droplets collide with neutral air molecules in the vicinity imparting momentum to the air molecules, thereby inducing an air flow. The induced air flow assists the charged droplets in moving away from the wick tip in the shape of a plume. In this example the electrode 10 is in the form of a toroid. This is preferred because it allows the aerosol plume to pass through it without affecting the shape of the plume unduly. Although a flat plate electrode would serve the purpose of providing a reference point, the plume would be diverted.

The polarity of the charge imparted can be either positive or negative according to requirements. The electrical supply can be mains or battery and can be selected to achieve different release rates of liquid pheromone. The porosity of the wick 7 can also be selected to achieve different release rates. The geometry of the wick 7 can be modified to direct the spray in any desired direction. The wick may consist of a single capillary or a device can be constructed with a series of separate wicks. The voltage may also be adjusted to maintain a liquid meniscus surface of constant diameter at the tip of the wick, without aerosol production, which would be a means of controlling the release rate of the liquid pheromone within precise limits.

The aerosol may also be electrically charged by means of a natural charge exchange phenomenon occurring during normal valve decompression in pressurised aerosol containers. This charge may be controlled by modification of the valve design and/or formulation of the product so as to enhance charge exchange. Once charged, the aerosol would then have the same dust removal characteristics as the purely electrostatic atomising system described above. The invention is particularly useful with relatively involatile pheromones.

Figure 2 shows an example of insect trapping apparatus according to the invention. A conventional electrical fly killer 11 consisting of an electrical grid 12 over strip lights 13 is provided with a distribution device 14 from which a plume 15 of pheromone attractant is emitted and dispersed by the induced air flow generated by the device itself, as described above.

Thus, by using the device of the present invention, the pheromone attractant is carried so that insects are drawn towards the fly killer from a distance rendering the fly killer more efficient in removing flies. The principle of using the distribution device in combination with a conventional trap can clearly be applied to other form of trap and other insects if the correct attractant is used.

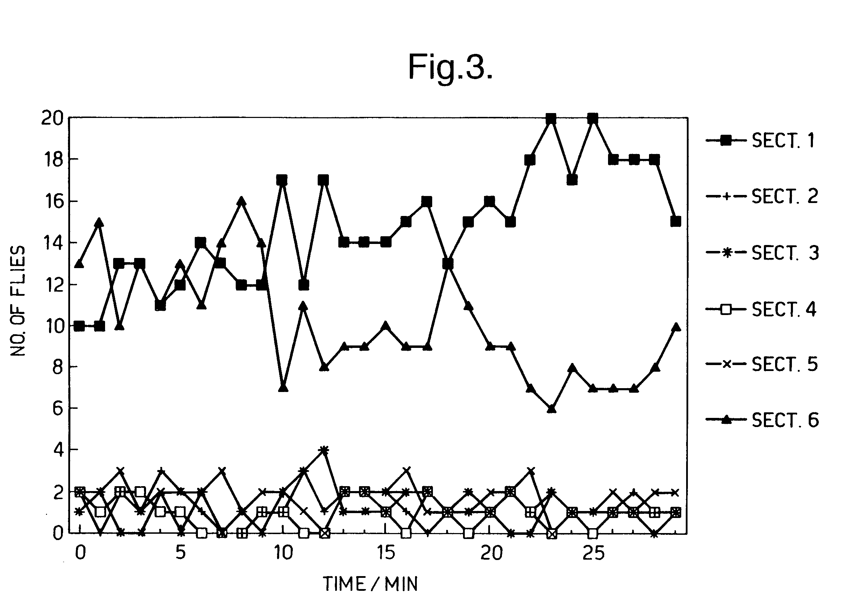

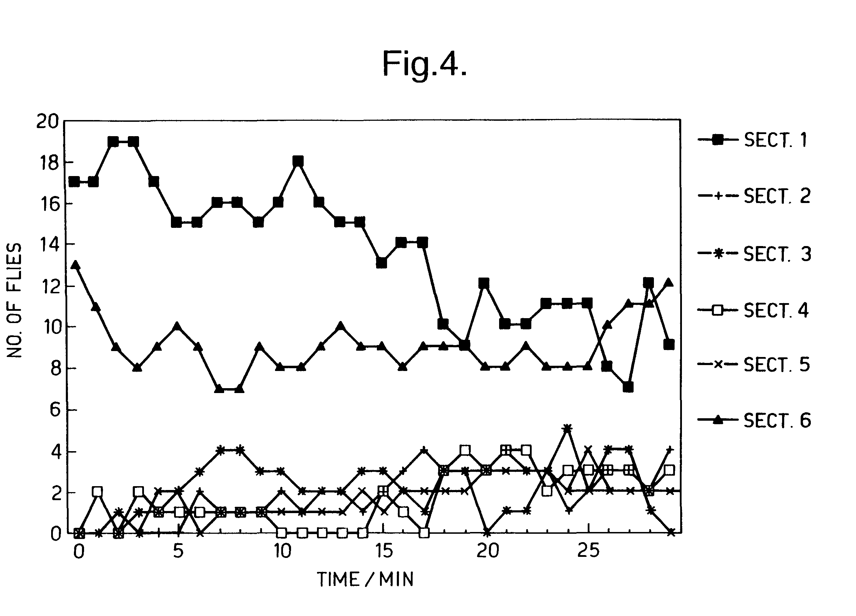

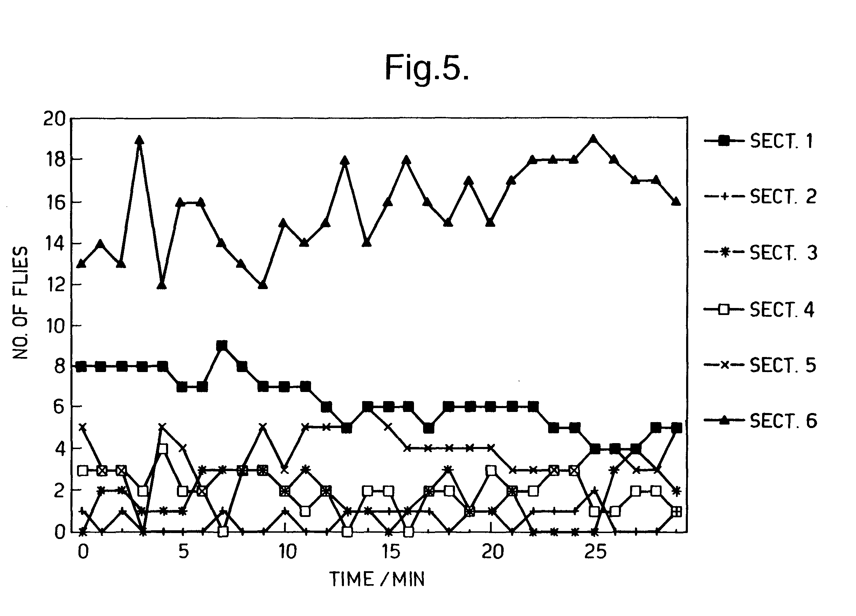

An example of experiments into the effect on the distribution of houseflies in a particular area for given conditions is described below. The conditions were
(i) no attractant used (Figure 3);
(ii) attractant used and distributed by conventional means (Figure 4); and
(iii) attractant used and distributed using the device of the present invention (Figure 5)

The experimental data was obtained by releasing 30 houseflies into a wind tunnel having dimensions of 1.2m long and 28cm in diameter. The tunnel was further sub-divided into six sectors of equal length where sector 6 was closest to a source of attractant and sector 1 was furthest from it and downwind.

Figure 3 shows the varying distribution of the houseflies over a 30 minute period with no attractant being emitted from the source and air passing from sector 6 to sector 1 at a speed of 0.1 ms⁻¹. The number of flies close to the source (sector 6) has reduced and the number of flies furthest from the source (sector 1) has increased so that at the end of the 30 minute period the majority of flies are in sector 1.

Figure 4 shows how the distribution varies if an attractant is put into the air by conventional means. In this case the number of flies in sector 1 tends to decrease and the number of flies in sector 6 decreases more slowly over the first 25 minutes. Only at the end of the measured period does the number of flies in sector 6, i.e. close to the source, start to increase.

Finally, Figure 5 illustrates the effect on distribution of houseflies of introducing attractant using the device of the present invention in 10 second bursts every minute. From the outset the majority of flies are found in sector 6 and with time this increases, indicating the significant improvement in efficiency of introducing the attractant as an aerosol using an electrostatic technique.

## Claims

1. For distributing biologically active material into the atmosphere, a combination of an electrostatic distribution device and a biologically active material (1), wherein:
the device comprises means (7,10) arranged to vapourise, volatilise or atomise the material (1) in aerosol form (9) using an electrostatic technique, whereby the so-vapourised, volatilised or atomised material (1) is suspended in the atmosphere; and
the biologically active material (1) is a pheromone.

2. A combination according to claim 1, wherein said means arranged to vapourise, volatilise or atomise the pheromone (1) in aerosol form (9) for subsequent atmospheric suspension, comprises means (7,10) arranged to subject the pheromone (1) to a sufficiently high electric field to cause particles of the pheromone (1) to become electrically charged to a sufficient extent to enable them to be released from the remainder of the pheromone (1) in aerosol form (9) into the atmosphere for subsequent suspension therein.

3. A combination according to claim 1 or 2, wherein the device further comprises a substrate (7) which is capable of having an electrical potential applied thereto and upon which the pheromone (1) to be vapourised, volatilised or atomised is receivable, such that particles of the pheromone (1) are electrically charged when subjected to an electric field generated by an electrical potential applied to the substrate (7).

4. A combination according to claim 3, wherein the substrate (7) is in the form of a surface to which the pheromone (1) to be vapourised, volatilised or atomised is suppliable.

5. A combination according to claim 4, wherein the surface is pointed to enhance the electric field at its tip, when the electrical potential is applied.

6. A combination according to claim 5, wherein the pointed substrate surface comprises one of an absorbent and a porous material.

7. A combination according to any of claims 3 to 6, wherein the substrate (7) is electrically conductive.

8. A combination according to any preceding claim, wherein the pheronome (1) to be vapourised, volatilised or atomised is subject directly to the electric field, whether or not any substrate (7) is used.

9. Insect trapping apparatus comprising a combination according to any of claims 1 to 8 and trapping means (11) arranged to trap insects lured by the pheromone (1).

10. A method of distributing biologically active material (1) into the atmosphere comprising vapourising, volatilising or atomising the material (1) in aerosol form (9) using an electrostatic technique, whereby the so-vapourised, volatilised or atomised material (9) is suspended in the atmosphere,
characterised in that the material (1) is a pheromone.

11. A method according to claim 10, wherein the electrostatic technique for vapourising, volatilising or atomising the pheromone (1) in aerosol form (9) for subsequent atmospheric suspension, comprises subjecting the pheromone (I) to a sufficiently high electric field to cause particles of the pheromone (1) to become electrically charged to a sufficient extent to enable them to be released from the remainder of the pheromone (1) in aerosol form (9) into the atmosphere for subsequent suspension therein.

12. A method of trapping insects comprising distributing a pheromone (1) into the atmosphere using a method according to claim 10 or 11, wherein a plume (14) of pheromone (1) is produced along which insects orientate; and providing trapping means (11) at the source of the plume (14), whereby insects are attracted by the pheromone (1) and follow the plume (14) to the trapping means (11) to be trapped.

## Patentansprüche

1. Kombination einer elektrostatischen Verteilungsvorrichtung und eines biologisch aktiven Materials (1), zum Verteilen von biologisch aktivem Material in die Atmosphäre, wobei:
die Vorrichtung eine Einrichtung (7,10) aufweist, die dazu ausgebildet ist, das Material (1) in Aerosol-Form (9) mittels einer elektrostatischen Technik zu verdampfen, verflüchtigen oder zerstäuben, wodurch das dergestalt verdampfte, verflüchtigte oder zerstäubte Material (1) in der Atmosphäre suspendiert wird; und
das biologisch aktive Material (1) ein Pheromon ist.

2. Kombination nach Anspruch 1, wobei die zum Verdampfen, Verflüchtigen oder Zerstäuben des Pheromons (1) in Aerosol-Form (9) für die nachfolgende atmosphärische Suspendierung ausgebildete Einrichtung eine Einrichtung (7,10) aufweist, die so ausgebildet ist, daß sie das Pheromon (1) einem ausreichend starken elektrischen Feld aussetzt, um zu bewirken, daß Partikel des Pheromons (1) in ausreichendem Maß elektrisch geladen werden, damit sie von dem restlichen Pheromon (1) in Aerosol-Form (9) in die Atmosphäre zur nachfolgenden Suspendierung in dieser freigesetzt werden.

3. Kombination nach Anspruch 1 oder 2, wobei die Vorrichtung weiterhin ein Substrat (7) aufweist, welches in der Lage ist, ein elektrisches Potential angelegt zu erhalten und an welchem das zu verdampfende, zu verflüchtigende oder zu zerstäubende Pheromon (1) aufgenommen werden kann, so daß Partikel des Pheromons (1) elektrisch geladen werden, wenn sie einem elektrischen Feld ausgesetzt werden, welches durch ein an das Substrat (7) angelegtes elektrisches Potential erzeugt wird.

4. Kombination nach Anspruch 3, wobei das Substrat (7) in Form einer Oberfläche ist, welcher das zu verdampfende, zu verflüchtigende oder zu zerstäubende Pheromon (1) zugeführt werden kann.

5. Kombination nach Anspruch 4, wobei die Oberfläche spitz ist, um das elektrische Feld an ihrer Spitze zu verstärken, wenn das elektrische Potential angelegt ist.

6. Kombination nach Anspruch 5, wobei die spitze Substratfläche entweder ein absorbierendes oder ein poröses Material aufweist.

7. Kombination nach einem der Ansprüche 3 bis 6, wobei das Substrat (7) elektrisch leitend ist.

8. Kombination nach einem der vorhergehenden Ansprüche, wobei das zu verdampfende, verflüchtigende oder zu zerstäubende Pheromon (1) dem elektrischen Feld direkt ausgesetzt wird, unabhängig davon, ob ein Substrat (7) verwendet wird oder nicht.

9. Insektenfalle, die aufweist: eine Kombination nach einem der Ansprüche 1 bis 8, und eine Falleneinrichtung (11), die so ausgebildet ist, daß sie durch das Pheromon (1) angelockte Insekten fängt.

10. Verfahren zum Verteilen von biologisch aktivem Material (1) in die Atmosphäre mit Verdampfen, Verflüchtigen oder Zerstäuben des Materials (1) in Aerosol-Form (9) mittels einer elektrostatischen Technik, wodurch das dergestalt verdampfte, verflüchtigte oder zerstäubende Material (9) in der Atmosphäre suspendiert wird,
dadurch gekennzeichnet, daß das Material (1) ein Pheromon ist.

11. Verfahren nach Anspruch 10, wobei die elektrostatische Technik zum Verdampfen, Verflüchtigen oder Zerstäuben des Pheromons (1) in Aerosol-Form (9) zur nachfolgenden Suspendierung in der Atmosphäre beinhaltet: Aussetzen des Pheromons (1) an ein ausreichend starkes elektrisches Feld, um zu bewirken, daß die Partikel des Pheromons (1) in einem ausreichenden Maß elektrisch geladen werden, daß sie von dem Rest des Pheromons (1) in Aerosol-Form (9) in die Atmosphäre für die anschließende Suspendierung in dieser feigegeben werden können.

12. Verfahren zum Fangen von Insekten, das aufweist: Verteilen eines Pheromons (1) in die Atmosphäre mittels eines Verfahrens nach Anspruch 10 oder 11, wobei eine Fahne (14) aus Pheromon (1) erzeugt wird, entlang welcher sich Insekten orientieren; und Bereitstellen einer Falleneinrichtung (11) an der Quelle der Fahne (14), wodurch Insekten von dem Pheromon (1) angezogen werden und der Fahne (14) zu der Falleneinrichtung (11) folgen, um dort gefangen zu werden.

## Revendications

1. Combinaison d'un dispositif de distribution électrostatique et d'une substance biologiquement active (1), qui est destinée à diffuser dans l'atmosphère des substances biologiquement actives, dans laquelle :
le dispositif comprend des moyens (7, 10) prévus pour vaporiser, volatiliser ou atomiser la substance (1) sous forme d'aérosol (9), en utilisant une technique électrostatique de façon à suspendre dans l'atmosphère la substance ainsi vaporisée, volatilisée ou atomisée, et
la substance biologiquement active (1) est un phéromone.

2. Combinaison suivant la revendication 1, dans laquelle les moyens prévus pour vaporiser, volatiliser ou atomiser le phéromone (1) sous forme d'aérosol 9), en vue d'une suspension atmosphérique, comprennent des moyens (7, 10) prévus pour soumettre le phéromone (1) à un champ électrique assez élevé pour faire que des particules du phéromone (1) se chargent électriquement jusqu'à un point suffisant pour pouvoir être libérées du reste du phéromone (1) sous forme d'aérosol (9) dans l'atmosphère pour y être ensuite suspendues.

3. Combinaison suivant la revendication 1 ou 2, dans laquelle le dispositif comprend, de plus, un substrat (7) à qui on peut appliquer un potentiel électrique et sur lequel on peut recevoir le phéromone (1) à vaporiser, volatiliser ou atomiser, de façon que des particules du phéromone soient chargées électriquement quand on le soumet à un champ électrique engendré par un potentiel électrique appliqué au substrat (7).

4. Combinaison suivant la revendication 3, dans laquelle le substrat (7) est sous la forme d'une surface sur laquelle on peut fournir le phéromone (1) à vaporiser, volatiliser ou atomiser.

5. Combinaison suivant la revendication 4, dans laquelle la surface est pointée pour augmenter le champ électrique à son sommet, quand on applique le potentiel électrique.

6. Combinaison suivant la revendication 5, dans laquelle la surface pointue du substrat comprend soit un matériau absorbant, soit un matériau poreux.

7. Combinaison suivant l'une quelconque des revendications 3 à 6, dans laquelle le substrat (7) est un conducteur électrique.

8. Combinaison suivant l'une des revendications précédentes, dans laquelle le phéromone (1) à vaporiser, volatiliser ou atomiser est soumis directement au champ électrique, que l'on utilise ou non un substrat (7).

9. Appareil à piéger les insectes comprenant une combinaison suivant l'une quelconque des revendications 1 à 8 et un moyen de piégeage (11) prévu pour piéger les insectes leurrés par le phéromone (1).

10. Méthode de diffusion de matériau actif biologiquement (1) dans l'atmosphère comprenant la vaporisation, la volatilisation ou l'atomisation du matériau (1) sous forme d'aérosol (9), en utilisant un technique électrostatique de façon que soit suspendu dans l'atmosphère le matériau (9) à vaporiser, volatiliser ou atomiser.
caractérisée en ce que le matériau (1) est un phéromone.

11. Méthode suivant la revendication 10, dans laquelle la technique électrostatique pour vaporiser, volatiliser ou atomiser le phéromone (1) sous forme d'aérosol 9), en vue d'une suspension atmosphérique, comprend la soumission du phéromone (1) à un champ électrique assez élevé pour faire que des particules du phéromone (1) deviennent chargées électriquement jusqu'à un point suffisant pour pouvoir être libérées du reste du phéromone (1) pour être ensuite suspendues dans l'atmosphère sous forme d'aérosol (9).

12. Méthode de piégeage des insectes comprenant la diffusion d'un phéromone (1) dans l'atmosphère en utilisant une méthode suivant la revendication 10 ou 11, dans laquelle un panache (14) du phéromone (1) est produit le long duquel s'orientent les insectes ; et en prévoyant un moyen de piégeage (11) à la source du panache (14), de façon que les insectes soient attirés par le phéromone (1) et suivent le panache (14) vers le moyen de piégeage (11) pour y être piégés.
